# EUROPÄISCHE PATENTSCHRIFT

(11) **EP 0 598 228 B1**
(45) Veröffentlichungstag und Bekanntmachung des Hinweises auf die Patenterteilung: **08.05.1996**
(21) Anmeldenummer: 93116791.0
(22) Anmeldetag: 18.10.1993
(51) Int. Cl.: C07C 29/141, C07C 29/145, C07C 31/20, C07C 45/52, C07C 45/64

(54) **Verfahren zur Herstellung von 1,2- und 1,3-Propandiol**
Method of preparation of 1,2- and 1,3- propanediol
Procédé pour la préparation de 1,2- et 1,3- propanediol

(30) Priorität: 14.11.1992 DE 4238492
(43) Veröffentlichungstag der Anmeldung: 25.05.1994
(73) Patentinhaber: Degussa Aktiengesellschaft, D-60311 Frankfurt (DE)
(72) Erfinder: Haas, Thomas,Dr., D-60316 Frankfurt (DE); Neher, Armin, Dr., D-63636 Brachttal (DE); Arntz, Dietrich, Dr., D-61440 Oberursel (DE); Klenk, Herbert, Dr., D-63457 Hanau (DE); Girke, Walter, D-63457 Hanau (DE)

(56) Entgegenhaltungen:
- EP-A- 0 487 903
- DE-C- 888 096
- FR-A- 695 931
- US-A- 2 042 224
- US-A- 2 558 520

## Beschreibung

Die Erfindung betrifft ein Verfahren zur gleichzeitigen Herstellung von 1,2- und 1,3-Propandiol aus einem Rohstoff, nämlich Glycerin, worin der erste Schritt eine Dehydratisierung von Glycerin darstellt.

1,2-Propandiol ist ein großtechnisch hergestelltes Produkt mit vielseitigem Einsatzspektrum. 1,3-Propandiol gewinnt Bedeutung als Baustein für Polyester. Zur Herstellung von 1,2- und 1,3-Propandiol sind unterschiedliche Verfahren bekannt.

1,2-Propandiol wird beispielsweise durch Hydrolyse von Propylenoxid gewonnen. 1,3-Propandiol wird im allgemeinen durch Hydratisierung von Acrolein an einem sauren Katalysator und katalytische Hydrierung des entstandenen 3-Hydroxypropionaldehyds gewonnen. Bezüglich der Herstellung von 1,3-Propandiol aus Acrolein wird beispielhaft auf die in den nachfolgenden Dokumenten beschriebenen Verfahren und den dort gewürdigten Stand der Technik verwiesen: DE-A 39 26 136, DE-A 40 38 192 sowie die DE-Patentanmeldungen P 41 38 982.4, P 42 18 282.4, P 41 38 981.6.

Es sind nur wenige Verfahren bekannt geworden, wonach aus einem Rohstoff 1,2- und 1,3-Propandiol gleichzeitig hergestellt werden:
Durch Umsetzung von Glycerin mit Kohlenmonoxid und Wasserstoff in einem organischen Lösungsmittel in Gegenwart eines homogenen Katalysatorsystems aus beispielsweise Wolframsäure und einer Rhodiumverbindung laßt sich 1,3- und 1,2-Propandiol gewinnen - siehe US-PS 4,642,394. Nachteilig an diesem Verfahren sind die niedrigen Ausbeuten an 1,2- und 1,3-Propandiol, welche jeweils 20 % kaum übersteigen; zudem muß Glycerin in einem Amin oder Amid gelöst umgesetzt werden, so daß sich wäßrige Glycerinlösungen nicht einsetzen lassen.

1,3-Propandiol läßt sich fermentativ aus Glycerin mit Clostridium butyricum herstellen - siehe B. Günzel et al., Applied Microbiology and Biotechnology, Springer Verlag 1991, Seiten 289-294. Obgleich dieser Weg zu 1,3-Propandiol in mittleren Ausbeuten (ca. 60 %) führt, darf nicht unberücksichtigt bleiben, daß die Raum-Zeit-Ausbeute an 1,3-Propandiol bei dieser Fermantation sehr gering, nämlich 2,3 bis 2,9 g·l⁻¹·h⁻¹, ist. Die Konzentration an Glycerin (um 5 bis 6 g/l) in der zu fermentierenden Lösung muß niedrig gehalten werden, da es ansonsten zur Inhibierung des Wachstums der eingesetzten Kulturen kommt; die Gewinnung des 1,3-Propandiols aus den sehr verdünnten Fermentationslösungen ist daher energieintensiv. 1,2-Propandiol wird nicht gebildet.

Gemäß einem weiteren mikrobiologischen Verfahren - Environ Microbiol. 46 (1), 62-67 (1983); Chem. Abstr. 99 (13):103663s - läßt sich Glycerin mittels Klebsiella pneumoniae NRRL B-199 in 55 %iger Ausbeute in 3-Hydroxypropionaldehyd, das Hydratationsprodukt von Acrolein, überführen, das in bekannter Weise zu 1,3-Propandiol hydriert werden kann. Die Fermentation erfolgt unter Einsatz etwa 3 %iger Glycerinlösungen und erfordert die Anwesenheit von Semicarbazidhydrochlorid, das wieder zurückgewonnen werden muß. Die Raum-Zeit-Ausbeute dieses Verfahrens ist gering; zusätzlich ist der gesamte Verfahrensaufwand groß. Offensichtlich wird kein Hydroxyaceton gebildet, das in 1,2-Propandiol überführt werden könnte.

Untersucht wurde zum Beispiel die Bildung von Acrolein aus Glycerin in der Gasphase unter Bedingungen der Reaktionsgaschromatographie (Ishikawa Koichi et al., Bunseki Kagaku 32 (10) E 321 - E 325, wobei eine sehr verdünnte wäßrige Glycerinlösung (1,5 - 150 mg/l) bei 260 bis 300 °C in gepulster Form über eine mit 10 bis 30 % KHSO₄ beschichtete Gaschrom R-Säule geleitet wird. Ein Fachmann erhält aus diesem Dokument keine Anregung, das Analysenverfahren zur Grundlage eines technischen Verfahrens zur Herstellung von 1,2- und 1,3-Propandiol aus Glycerin zu machen, weil als Dehydratisierungsprodukt nur Acrolein genannt wird und zudem die extreme Verdünnung völlig andere Verhältnisse schafft als Verdünnungen, wie sie in technischen Verfahren notwendig sind, um eine befriedigende Raum-Zeit-Ausbeute und Katalysator-Standzeit zu erzielen.

Aus der FR-PS 695 931 ist ein Verfahren zur Herstellung von Acrolein aus Glycerin bekannt, wobei Glycerindämpfe bei über 300 °C, inbesondere 400 bis 420 °C, über einen Festbettkatalysator geleitet werden. Als Katalysator werden Salze dreibasiger Säuren oder Mischungen solcher Salze, welche sich auf auf einem Träger befinden können, beansprucht; ausweislich der Beispiele wird mit 1 % Lithiumphosphat beziehungsweise 1 % Eisenphosphat belegter Bimsstein eingesetzt. In diesem Dokument wird die Acrolein-Ausbeute ausweislich der Beispiele mit 75 % beziehungsweise 80 % angegeben. Hinweise, das Reaktionsgemisch der Dehydratisierung unmittelbar zur Herstellung von 1,2- und 1,3-Propandiol zu verwenden, lassen sich diesem Dokument nicht entnehmen. Die Erfinder der vorliegenden Patentanmeldung haben das Verfahren der FR-PS 695,931 nachgearbeitet und dabei festgestellt, daß unter den getesteten Reaktionsbedingungen weder mit Lithiumphosphat noch mit Eisenphosphat die ausgewiesenen Ausbeuten erhalten werden konnten: Wie die Vergleichsbeispiele zeigen, lag bei 300 °C die Ausbeute an Acrolein nur bei etwa 1 bis 3 % und bei 400 °C bei 30 bis 35 %.

Aufgabe der Erfindung ist, ein technisches Verfahren zur gleichzeitigen Herstellung von 1,2- und 1,3-Propandiol aus Glycerin aufzuzeigen, das nicht-fermentativ ist und die Propandiole in höherer Gesamtausbeute herzustellen gestattet, als dies im vorbekannten Verfahren möglich war. Zusätzlich sollte sich die Verwendung organischer Lösungsmittel erübrigen.

Die Aufgabe wird durch ein Verfahren gelöst, das dadurch gekennzeichnet ist, daß es die Reaktionsstufen
(a) Dehydratisierung von Glycerin unter Bildung einer Acrolein und Hydroxyaceton enthaltenden wäßrigen Lösung, indem ein Glycerin-Wasser-Gemisch mit einem Glyceringehalt von 10 bis 40 Gew.-% in der Gasphase bei 250 bis 340 °C über einen sauren Feststoffkatalysator mit einem H₀-Wert (Hammett'sche Säurefunktion) kleiner +2 geleitet und der gasförmige Produktstrom zu der genannten wäßrigen Lösung kondensiert wird,
(b) Hydratisierung des im kondensierten Produktstrom enthaltenen Acroleins zu 3-Hydroxypropionaldehyd durch Behandlung des kondensierten Produktstroms der Stufe (a) in an sich bekannter Weise bei 20 bis 120 °C in Gegenwart eines üblichen sauren Hydratisierungskatalysators und
(c) katalytische Hydrierung des in der wäßrigen Reaktionslösung der Stufe (b) enthaltenen 3-Hydroxypropionaldehyds und Hydroxyacetons zu 1,3-und 1,2-Propandiol, indem die Reaktionslösung der Stufe (b) von nicht umgesetztem Acrolein befreit und anschließend in an sich bekannter Weise unter Verwendung üblicher Hydrierkatalysatoren hydriert wird, und destillative Auftrennung des Reaktionsgemischs der Stufe (c)
umfaßt.

Es wurde gefunden, daß bei der Dehydratisierung von Glycerin unter den anspruchsgemäßen Bedingungen (Reaktionsstufe (a)) außer Acrolein auch Hydroxyaceton gebildet wird. Erfindungsgemäß wird der Produktstrom der Stufe (a) ohne vorherige Reinigung in der Reaktionsstufe (b) solchen Bedingungen unterworfen, unter welchen Acrolein in an sich bekannter Weise in wäßriger Phase in 3-Hydroxypropionaldehyd überführt wird. Der Produktstrom der Stufe (b), welcher 3-Hydroxypropionaldehyd und Hydroxyaceton enthält, wird nach Abtoppen oder Abdestillieren nicht umgesetzten Acroleins der Hydrierstufe zugeführt, wobei die vorgenannten Carbonylverbindungen unter üblichen Bedingungen, wie sie insbesondere aus der Hydrierung von 3-Hydroxypropionaldehyd zu 1,3-Propandiol bekannt sind, zu 1,3- und 1,2-Propandiol hydriert werden.

Es war nicht vorhersehbar, daß 1,2- und 1,3-Propandiol gleichzeitig aus Glycerin in guter Gesamtausbeute erhalten werden konnten. Überraschend war darüber hinaus, daß es mögich war, beide Wertprodukte erst nach der dritten Reaktionsstufe voneinander zu trennen; es bedurfte somit keiner Zwischenreinigung des Produktstroms auf der Stufe (a) und der Stufe (b), ausgenommen der Abtrennung gegebenenfalls nicht umgesetzten Acroleins vor der Stufe (c). Schließlich war es im Hinblick auf den Stand der Technik nicht zu erwarten, daß kein organisches Lösungsmittel erforderlich war, vielmehr eine wäßrige Glycerinlösung als Ausgangsprodukt eingesetzt werden konnte.

Erfindungswesentlich ist, daß wäßrige Glycerinlösung mit einer Konzentration zwischen 10 und 40 Gew.-%, vorzugsweise zwischen 10 und 25 Gew.-%, über den festen Dehydratisierungskatalysator geleitet wird. Damit lassen sich sogenannte Rohglycerine aus der Fettspaltung einsetzen, ohne daß Glycerin zunächst konzentriert und gereinigt werden muß. Zwar kommt es bei der Verwendung einer Glycerinlösung mit einem Gehalt oberhalb 40 Gew.-% noch zur Dehydratisierung, jedoch sinken mit steigender Glycerinkonzentration sowohl die Selektivität der Umsetzung als auch die Standzeit des Katalysators merklich ab. Die sinkende Selektivität macht sich insbesondere durch einen wachsenden Hochsiederanteil bemerkbar. Wäßriges Glycerin mit einer Konzentration unter 10 Gew.-% läßt sich zwar einsetzen, jedoch wird die Wirtschaftlichkeit des Verfahrens mit abnehmender Konzentration gemindert, da die Raum-Zeit-Ausbeute abnimmt und der Energieaufwand bei der destillierten Auftrennung des Reaktionsgemisch der Stufe (c) zunimmt.

Die Umsetzung des Glycerins in der Gasphase führt im allgemeinen zu einem Glycerinumsatz von praktisch 100 % und unter den anspruchsgemäßen Bedingungen zu einer hohen Selektivität bezüglich der Bildung von Acrolein und Hydroxyaceton. Das den Katalysator verlassende gasförmige Reaktionsgemisch (=Produktstrom), welcher außer Acrolein und Hydroxyaceton zusätzlich gebildete Nebenprodukte enthält, wird unmittelbar kondensiert und der nächsten Stufe zugeführt.

Als saure Feststoffkatalysatoren werden feste, unter den Reaktionsbedingungen im wesentlichen stabile ein-oder mehrphasig aufgebaute Stoffe mit einem Hₒ-Wert kleiner +2, vorzugsweise kleiner -3, eingesetzt. Der Hₒ-Wert entspricht der Säurefunktion nach Hammett und läßt sich durch die sogenannte Amintitration unter Verwendung von Indikationen oder durch Adsorption einer gasförmigen Base ermitteln - siehe Studies in surface science and catalysis, Vol. 51, 1989: "New solid acids and bases, their catalytic properties" by K. Tanabe et al. Kapitel 2, insbesondere Seiten 5-9. Kapitel 1 des vorgenannten Dokuments nennt auf den Seiten 1-3 zahlreiche feste Säuren, aus welchen der Fachmann, gegebenenfalls nach Bestimmung des Hₒ-Wertes, den geeigneten Katalysator auswählen kann. Als Feststoffkatalysatoren für die Dehydratisierung eignen sich beispielsweise (i) natürliche und synthetische silikatische Stoffe, wie insbesondere Mordenit, Montmorillonit, saure Zeolithe; (ii) mit ein-, zwei- oder mehrbasigen anorganischen Säuren, insbesondere Phosphorsäure, oder sauren Salzen anorganischer Säuren belegte Trägerstoffe, wie oxidische oder silikatische Stoffe, beispielsweise Al₂O₃, TiO₂; (iii) Oxide und Mischoxide, wie beispielsweise gamma-Al₂O₃ und ZnO-Al₂O₃-Mischoxide oder Heteropolysäuren.

Für die Dehydratisierung in der Gasphase werden Katalysatoren mit einem Hₒ-Wert zwischen -3 und -8,2 besonders bevorzugt; hierzu zählt H₃PO₄ auf Al₂O₃, sogenannte feste Phosphorsäure. Katalysatoren vom Typ Zeolith-H-ZSM5 sind wegen ihres Hₒ-Wertes von kleiner -8,2 für die Gasphasendehydratisierung weniger geeignet.

Die Herstellung der Katalysatoren vom Typ (ii) ist besonders einfach: Das Trägermaterial wird mit einer wäßrigen Lösung der Säure behandelt und der so behandelte Feststoff getrocknet.

Die Dehydratisierung von Glycerin in der Gasphase erfolgt bei 250 bis 340 °C, vorzugsweise im Temperaturbereich oberhalb 270 bis 320 °C. Eine Steigerung der Temperatur auf über 340 °C bewirkt eine deutliche Abnahme der Selektivität. Überraschenderweise wurde gefunden, daß durch eine Begrenzung der Temperatur in der Dehydratisierungsstufe auf maximal 340 °C und vorzugsweise 320 °C die Standzeit der Katalysatoren derart gesteigert wird, daß sie im Dauerbetrieb im technischen Maßstab eingesetzt werden können.

Die erfindungsgemäße Reaktionsstufe (a) kann in üblichen Apparaturen, wie sie dem Fachmann für Gasphasenreaktionen an einem Feststoffkatalysator geläufig sind, durchgeführt werden. Im wesentlichen umfaßt eine solche Apparatur einen Verdampfer für das Glycerin-Wasser-Gemisch, den Reaktor, in welchem der Katalysator in der Regel in Form eines Festbetts angeordnet ist und der Heizvorrichtungen für eine oder mehrere Temperaturzonen enthält, sowie eine Vorrichtung zur Kondensierung des gasförmigen Produktstromes.

Daß sich Glycerin in der Gasphase in Gegenwart fester saurer Katalysatoren unter Bildung von Acrolein dehydratisieren läßt, war zwar bekannt, aber erst durch die anspruchsgemäßen Bedingungen wurde es möglich, die Reaktionsstufe (a) so durchzuführen, daß daraus eine hohe Gesamtausbeute an den beiden gewünschten Dehydratisierungsprodukten, eine hohe Raum-Zeit-Ausbeute und eine gute Standzeit des Katalysators resultierten.

Die Reaktionsstufe (b) des erfindungsgemäßen Verfahrens wird in gleicher Weise durchgeführt, wie sie aus Verfahren zur Hydratisierung von Acrolein in wäßrigen Lösungen bekannt ist. Der einzige Unterschied zu den bekannten Verfahren zur Herstellung von 3-Hydroxypropionaldehyd aus Acrolein besteht darin, daß es sich bei der einzusetzenden wäßrigen Acroleinlösung um das kondensierte Reaktionsgemisch der Stufe (a), welches zusätzlich Hydroxyaceton enthält, handelt. Die Hydratisierung erfolgt in Gegenwart eines sauren Katalysators, entweder in homogener Phase, beispielsweise mittels eines Säure-Base-Puffers bei pH 3 bis 4,5 gemäß der DE-Patentanmeldung P 41 38 981.6, oder in heterogener Phase, beispielsweise mittels eines Kationenaustauschers mit chelatbildenden Ankergruppen gemäß der DE-A 39 26 136 oder DE-A 40 38 192 oder mittels einer trägergebundenen Säure gemäß DE-Patentanmeldung P 41 38 982.4, vorzugsweise bei einer Temperatur im Bereich von 50 bis 90 °C und einem Druck von 1 bis 20 bar. Im Anschluß an die Hydratisierung wird das Reaktionsgemisch von nicht umgesetztem Acrolein befreit, etwa durch Strippen oder Abdestillieren; das Acrolein wird erneut der Hydratisierung zugeführt.

Sofern erwünscht, kann aus dem Reaktionsgemisch der Stufe (b) während oder nach der Acroleinabtrennung zusätzlich ein Teil des anwesenden Wassers abdestilliert werden.

Das vom Acrolein befreite Reaktionsgemisch der Stufe (b) wird in der Reaktionsstufe (c) in an sich für Ketone und Aldehyde bekannter Weise unter Verwendung üblicher Hydrierkatalysatoren mit Wasserstoff hydriert. Vorzugsweise wird in Gegenwart eines Festbett- oder Suspensionskatalysators bei 5 bis 300 bar, einem pH-Wert von 2,5 bis 6,5 und einer Temperatur von 30 bis 180 °C hydriert; besonders zweckmäßig ist es, die Hydrierung gemäß der DE-Patentanmeldung P 42 18 282.4 zunächst bei 30 bis 80 °C und dann bei 100 bis 180 °C durchzuführen. Unter üblichen Katalysatoren werden solche verstanden, wie sie beispielsweise in der US 2,434,110 , DE-OS 39 26 136 und DE-OS 40 38 192 genannt werden. Bevorzugt werden Katalysatoren aus der Reihe
i) Raney-Nickel, das auch mit anderen hydrierwirksamen Metallen dotiert sein kann, soweit es sich ausschließlich um eine Suspensionshydrierung handelt,
ii) Trägerkatalysatoren auf der Basis platin- oder rutheniumbeschichteter Aktivkohle oder Metalloxide, wie insbesondere Al₂O₃, SiO₂ oder TiO₂
   oder
iii) nickelbeschichteter oxidischer oder silikatischer Trägerkatalysatoren, vorzugsweise Katalysatoren auf der Basis von Ni/Al₂O₃/SiO₂.

Vorteile des erfindungsgemäßen Verfahrens sind: gleichzeitig sind zwei Wertstoffe, nämlich 1,2- und 1,3-Propandiol (PD), in hoher Gesamtausbeute aus Glycerin zugänglich; die Verwendbarkeit von sogenanntem Rohglycerin mit einem Gehalt von 15 bis 30 Gew.-% senkt die Rohstoffkosten; das Verfahren läßt sich mit hoher Selektivität und hoher Raum-Zeit-Ausbeute betreiben; es sind keine organischen Lösungsmittel erforderlich.

Das nachfolgende Beispiel verdeutlicht die Erfindung.

### Beispiel

100 g Rosenthal-Kugeln (Al₂O₃) mit einem Durchmesser von 3 mm werden mit 25 g einer 20 gew.-%igen Phosphorsäure 1 h gemischt. Am Rotationsverdampfer wird bei 80 °C das überschüssige Wasser abgezogen. 100 ml dieses Katalysators werden in ein Stahlrohr von 15 mm Durchmesser eingefüllt. Es wird eine 20 gew.-%ige wäßrige Glycerin-Lösung über eine Pumpe mit 40 ml/h in einen auf 300 °C beheizten Verdampfer gefördert und der Gasstrom direkt über den Katalysator bei 300 °C geleitet. Bei quantitativem Glycerin-Umsatz erhält man in dem kondensierten Produktstrom eine Ausbeute von 70,5 % Acrolein und ca. 10 % Hydroxyaceton, jeweils bezogen auf Glycerin.

Das resultierende gasförmige Reaktionsgemisch wird anschließend kondensiert. Zur Hydratisierung des Acroleins zu 3-Hydroxypropionaldehyd wird der kondensierte Produktstrom der Stufe (a) gemäß DE-A 40 38 192 an einem Ionenaustauscher mit Iminodiessigsäure-Ankergruppen (Lewatit TP 208, Fa. Bayer AG) umgesetzt. Das nicht umgesetzte Acrolein der Stufe (b) wird als 96 %iges Azeotrop mit H₂O destillativ vom Produktgemisch getrennt und dem Produktstrom der Stufe (a) zugeführt. Unter stationären Bedingungen erhält man eine Acroleinanfangskonzentration von 14,3 %; Reaktionstemperatur 50 °C; LHSV = 0,5 h⁻¹; Umsatz = 60 %, Selektivität = 85 %.

Das vom Acrolein befreite Reaktionsgemisch der Stufe (b) wird gemäß DE-Patentanmeldung P 42 18 282.4 hydriert. Hydrierbedingungen: Festbettkatalysator aus Ni/Al₂O₃/SiO₂; Temperatur der ersten Stufe 40 °C und der zweiten Stufe 140 °C; H₂-Druck 40 bar; LHSV 1 h⁻¹. Die hydrierte Reaktionslösung enthält 9,9 Gew.-% 1,3-Propandiol und 1,0 Gew.-% 1,2-Propandiol. Das Gemisch wird destillativ aufgearbeitet: Siedepunkte bei 50 mbar 109 °C für 1,2-PD und 134 °C für 1,3-PD. Die Produktqualität des 1,2- und des 1,3-Propandiols entspricht derjenigen, wie sie aus marktüblichen Produkten bekannt ist. Ausbeute an 1,3-PD, bezogen auf eingesetztes Glycerin, 60 %, jene an 1,2-PD 10 %.

## Patentansprüche

1. Verfahren zur gleichzeitigen Herstellung von 1,2- und 1,3-Propandiol aus Glycerin,
dadurch gekennzeichnet,
daß es die Reaktionsstufen
(a) Dehydratisierung von Glycerin unter Bildung einer Acrolein und Hydroxyaceton enthaltenden wäßrigen Lösung, indem ein Glycerin-Wasser-Gemisch mit einem Glyceringehalt von 10 bis 40 Gew.-% in der Gasphase bei 250 bis 340 °C über einen sauren Feststoffkatalysator mit einem H₀-Wert (Hammett'sche Säurefunktion) kleiner +2 geleitet und der gasförmige Produktstrom zu der genannten wäßrigen Lösung kondensiert wird,
(b) Hydratisierung des im kondensierten Produktstrom enthaltenen Acroleins zu 3-Hydroxypropionaldehyd durch Behandlung des kondensierten Produktstroms der Stufe (a) in an sich bekannter Weise bei 20 bis 120 °C in Gegenwart eines üblichen sauren Hydratisierungskatalysators und
(c) katalytische Hydrierung des in der wäßrigen Reaktionslösung der Stufe (b) enthaltenen 3-Hydroxypropionaldehyds und Hydroxyacetons zu 1,3- und 1,2-Propandiol, indem die Reaktionslösung der Stufe (b) von nicht umgesetztem Acrolein befreit und anschließend in an sich bekannter Weise unter Verwendung üblicher Hydrierkatalysatoren hydriert wird, und destillative Auftrennung des Reaktionsgemischs der Stufe (c)
umfaßt.

2. Verfahren nach Anspruch 1,
dadurch gekennzeichnet,
daß die Dehydratisierung (Stufe (a)) bei 270 bis 320 °C durchgeführt wird.

3. Verfahren nach Anspruch 1 oder 2,
dadurch gekennzeichnet,
daß man die Dehydratisierung unter Verwendung eines Feststoffkatalysators mit einem H₀-Wert zwischen -3 und -8,2 durchführt.

4. Verfahren nach Anspruch 3,
dadurch gekennzeichnet,
daß man als Feststoffkatalysator mit Phosphorsäure belegte anorganische Trägermaterialien, insbesondere Aluminiumoxid, verwendet.

## Claims

1. A process for the simultaneous production of 1,2- and 1,3-propanediol from glycerol,
characterised in that it comprises the reaction stages:
(a) dehydration of glycerol with formation of an aqueous solution containing acrolein and hydroxyacetone by leading a glycerol-water mixture with a glycerol content of 10 to 40 wt% in the gas phase at 250 to 340 °C over an acidic solid catalyst with an H₀ value (Hammett acidity function) of less than +2 and condensing the gaseous product stream to the aqueous solution mentioned,
(b) hydration of the acrolein contained in the condensed product stream to 3-hydroxypropionaldehyde by treatment of the condensed product stream of stage (a) in a way known per se at 20 to 120 °C in the presence of a conventional acidic hydration catalyst and
(c) catalytic hydrogenation of the 3-hydroxypropionaldehyde and hydroxyacetone contained in the aqueous reaction solution of stage (b) to 1,3- and 1,2-propanediol by freeing the reaction solution of stage (b) from unreacted acrolein and subsequently hydrogenating the reaction solution in a way known per se, using conventional hydrogenation catalysts, and splitting up the reaction mixture of stage (c) by distillation.

2. A process according to Claim 1,
characterised in that the dehydration (stage (a)) is carried out at 270 to 320 °C.

3. A process according to Claim 1 or 2,
characterised in that the dehydration is carried out with use of a solid catalyst with an H₀ value between -3 and -8.2.

4. A process according to Claim 3,
characterised in that inorganic support materials, in particular aluminium oxide, coated with phosphoric acid are used as the solid catalyst.

## Revendications

1. Procédé pour la préparation simultanée de 1,2- et 1,3-propanediol à partir de glycérol, caractérisé en ce qu'il comporte les étapes de réaction :
(a) la déshydratation de glycérol avec formation d'une solution aqueuse contenant de l'acroléine et de l'hydroxyacétone, en faisant passer un mélange eauglycérol ayant une teneur en glycérol de 10 à 40 % en poids dans la phase gazeuse à 250 jusqu'à 340°C sur un catalyseur solide ayant une valeur H_{O} (fonction acide de Hammett) plus petite que +2 et en condensant le courant de produit gazeux dans la solution aqueuse citée,
(b) l'hydratation de l'acroléine contenue dans le courant de produit condensé en 3-hydroxypropionaldéhyde par traitement du courant de produit condensé de l'étape (a) d'une manière connue en soi entre 20 et 120°C en présence d'un catalyseur d'hydratation acide usuel et,
(c) l'hydratation catalytique du 3-hydroxypropionaldéhyde et de l'hydroxyacétone contenu dans la solution réactionnelle aqueuse de l'étape (b) en 1,3- et 1,2-propanediol, tandis que la solution réactionnelle de l'étape (b) est libérée de l'acroléine non transformée et ensuite est hydrogénée d'une manière connue en soi en utilisant des catalyseurs d'hydrogénation usuels, et séparation par distillation du mélange réactionnel de l'étape (c).

2. Procédé selon la revendication 1, caractérisé en ce que la déshydratation (étape (a)) est réalisée de 270 à 320°C.

3. Procédé selon les revendications 1 ou 2, caractérisé en ce qu'on réalise la déshydratation en utilisant un catalyseur de matière solide ayant une valeur H_{O} comprise entre -3 et -8,2.

4. Procédé selon la revendication 3, caractérisé en ce qu'on utilise comme catalyseur de matière solide des matériaux de support minéraux recouverts d'acide phosphorique, en particulier de l'oxyde d'aluminium.
